# EUROPEAN PATENT APPLICATION

(11) **EP 4 797 271 A1**
(43) Date of publication of application: **26.08.2026**
(21) Application number: 25175332.3
(22) Date of filing: 09.05.2025
(51) Int. Cl.: G16H 10/60, G16H 20/10, G16H 50/20, G16H 50/70, G16H 70/20

(54) **METHOD AND COMPUTER PROGRAM PRODUCT FOR IMPROVING THE CARE OF ONCOLOGY PATIENTS**

(30) Priority: 20.02.2025 US 202563760747 P; 20.02.2025 EP 25159079
(71) Applicant: König, Frank, 12161 Berlin (DE)
(72) Inventor: König, Frank, 12161 Berlin (DE); König, Robin Ben, 12161 Berlin (DE)
(74) Representative: Hertin und Partner Rechts- und Patentanwälte PartG mbB

(57) **Abstract**

In a first aspect, the invention relates to a computer-implemented method for supporting a treatment of a cancer patient. The patient is provided with information about cancer treatment and related guidelines. During the treatment process, patient data is collected through a module via a user interface. This data is then pseudonymized to ensure privacy and stored on a server. The stored data is subsequently analysed, and based on this analysis, a treatment recommendation is provided at the user interface to assist in the patient's ongoing treatment.

In a further aspect, the invention relates to a computer program product.

## Description

In a first aspect, the invention relates to a computer-implemented method for supporting a treatment of a cancer patient. The patient is provided with information about cancer treatment and related guidelines. During the treatment process, patient data is collected through a module via a user interface. This data is then pseudonymized to ensure privacy and stored on a server. The stored data is subsequently analysed, and based on this analysis, a treatment recommendation is provided at the user interface to assist in the patient's ongoing treatment.

In a further aspect, the invention relates to a computer program product.

### Background and prior art

Despite groundbreaking research results in the treatment of malignant tumours, the implementation of the findings in the daily care of patients is inadequate. Although the new treatment strategies are quickly incorporated into national and international guidelines, state-of-the-art therapy is usually only carried out in specialized or certified oncology centres. Data on the proportion of cancer cases treated in accordance with guidelines is scarce. In total, around 500,000 new cases of cancer are diagnosed in Germany every year. Only around two thirds of all oncology patients are treated in certified structures, while others are not. According to a recent statement by the government commission for a modern and needs-based hospital reform, for example, only 39 percent of patients with lung cancer (57,000 new diagnoses per year) are treated in certified centres where care in line with guidelines can be expected. The situation is similar in the USA. There is no nationwide survey that determines the exact proportion of cancer patients treated according to guidelines for all cancer types and regions. Factors such as socioeconomic status, access to medical care and regional differences can influence adherence to guidelines.

In connection with inadequate guideline-compliant therapy, there is also a serious lack of psycho-oncological care for patients. There are no concrete figures on the need for and supply of psycho-oncological care. This is primarily because in Germany, for example, there is no billing option for psycho-oncological counselling services between doctors and health insurance companies in the outpatient sector. There is therefore also no nationwide use of questionnaires and evaluation forms on this topic.

There are numerous studies and projects dedicated to analysing the course of disease in oncology patients. However, these are usually limited in terms of time and location. An excellent example is the FLATIRON Clinico-Genomic Database (CGDB) project, which was initiated in 2012 by Nat Turner and Zach Weinberg (formerly employees at Google Inc.). The content was the recording and analysis of data from electronic patient records in 280 oncology facilities. A total of approx. 280,000 disease courses were documented, including approx. 10,000 prostate carcinomas (2021). The database also included around 60,000 gene profiles (Foundation Medicine Inc.). The database was sold by Flatiron Health to ROCHE in 2018 for USD 1.9 billion.

This example illustrates the economic value of such data in addition to the gain in scientific knowledge. The uniqueness of such data lies in the representation of the current care reality of tumour patients.

New forms of therapy for oncological diseases are increasing exponentially. There is an increasing shift from uniform systemic therapies (e.g. chemotherapy) to more and more personalized therapies (e.g. immunotherapies, antibody-based drug conjugates, etc.). Only by analysing large amounts of data will it be possible to compare the effectiveness of the numerous therapy options in the future. In addition, the detection of rare side effects of new drugs is only possible by recording as many disease courses as possible.

The guideline-based treatment of oncology patients is inadequate. A computer program product (such as a patient app) for individual counselling before selecting a specific treatment method and/or for support during the course of the disease could help to improve this situation.

### Objective of the invention

It is an objective of the invention to provide a method and a computer program product for supporting a patient during his or her cancer treatment.

### Summary of the invention

The problem according to the invention is solved by the independent claims. Advantageous embodiments of the invention are disclosed in the dependent claims.

In a first aspect, the invention relates to a computer-implemented method for supporting a treatment of a cancer patient comprising the following steps:
- Providing information on cancer treatment and cancer treatment guidelines to the cancer patient,
- collecting patient data on a module via a user interface during the treatment,
- pseudonymizing the patient data,
- storing the patient data on a server,
- analysing the patient data,
- providing a treatment recommendation at the user interface to support the treatment of the cancer patient.

The computer-implemented method according to the invention is advantageous in that it provides a constant, permanent, and competent companion who supports the patient during their cancer treatment. When referring to the method hereafter, it means the computer-implemented method in the context of the invention.

Cancer patients are generally not familiar with the disease in terms of medical expertise. Moreover, in addition to the physical damage, cancer patients also experience psychological effects, as the disease imposes extreme stress. The method according to the invention enables additional support for the patient, for example, by providing information about the disease itself and/or addressing the effects of specific treatment methods. This provides better support for the patient, which can also alleviate some of the psychological stress.

A particular advantage is that the method allows for adjusting the treatment of the cancer patient. In this respect, it is preferred that the treatment support involves adjusting the treatment to address the side effects the cancer patient is experiencing from the treatment.

Treatment adjustments can include, for example, dose reduction or pausing chemotherapy, changing therapy due to skin reactions from immunotherapy, switching medications in case of liver problems, changing from intravenous to oral chemotherapy due to nausea, modifying treatment due to circulatory issues and/or using supportive medication in case of anaemia.

It is known that chemotherapy can cause severe nausea, vomiting, and/or infections due to neutropenia. The chemotherapy dose can then be reduced or the treatment paused for several cycles to allow the body time to recover. For example, if oral chemotherapy causes less severe nausea, the treatment can be adjusted accordingly. In some cases, supportive medications, such as antiemetics or growth factors to support the immune system, are added. Immunotherapy can cause side effects like skin rashes, itching, and/or swelling. In such cases, the treatment may be interrupted or reduced to a lower dose. In some instances, symptomatic treatments such as corticosteroid creams are used. Increased liver enzymes or liver damage can be treated by adjusting the medication. This could involve dose reduction, a pause, or switching to a different drug that is less toxic to the liver. In cases of heart complications or cardiovascular issues, therapy may be changed or switched to a less cardiotoxic option to avoid further heart damage. If anaemia occurs, the treatment can be supplemented with erythropoietin (medications that stimulate blood formation), or the chemotherapy dose can be reduced to lessen the burden on the body.

The adjustments in treatment support aim to maximize the effectiveness of the treatment while maintaining the patient's quality of life by reducing side effects. Each adjustment is made individually, based on the severity of the side effects and the patient's overall health condition.

Another advantage is that the data is pseudonymized. This allows the data from the module to be analysed and researched without concerns regarding personal rights. The use of large data sets from cancer patients in cancer research is advantageous for developing new and better treatment approaches.

Large datasets containing information on genetic markers, mutations, and molecular profiles of tumours enable the development of specific treatment strategies that are better tailored to a patient's individual characteristics. By analysing DNA data, researchers can identify mutations in genes linked to certain types of cancer, leading to targeted therapies aimed at the specific genetic changes in a tumour. By collecting data on risk factors (e.g., lifestyle, family history, environmental factors), patterns can be discovered that help in early cancer detection. Large data sets from patients can also help to identify early biomarkers that signal the presence of cancer before symptoms appear. Data from many clinical studies can be used to determine the best treatment methods for different patient groups, including treatment outcomes, side effects, and survival rates. By analysing patient data, researchers can better understand which treatment protocols are most effective for specific cancer patients and how side effects can be minimized. Analysis of large data sets containing information on drugs, active substances, and their effects on tumours can help identify new potential drug candidates. Data from gene expression and proteomics can assist in identifying biomarkers that could serve as targets for new drugs. Data on different types of cancer and their progression helps in understanding cancer's origins, including genetic, epigenetic, and environmental factors contributing to tumour formation.

By collecting and analysing pseudonymized data, a deeper and more comprehensive insights into the various aspects of cancer can be gained. These data enable the improvement of existing treatment methods and the development of new therapies that are better tailored to the individual needs of patients.

It is preferred that information about cancer treatment and cancer treatment guidelines be provided to the patient.

This step involves providing basic information about cancer treatments and (general) treatment guidelines for cancer patients. This could be for example in the form of texts, conversations, videos, or interactive content that help the patient gain a better understanding of available treatment options, the underlying treatment recommendations, and the approach to cancer treatment. This provides the patient with fundamental knowledge so they can make informed decisions about their treatment. Cancer information includes, for example, details about different types of cancer, typical treatment methods (e.g., chemotherapy, radiation therapy, immunotherapy), potential side effects, and the frequency of certain procedures.

Cancer treatment guidelines are preferably standardized recommendations or protocols developed by professional societies and/or medical organizations. They are based on scientific evidence and aim to ensure that patients receive the best possible treatment based on their specific cancer type.

Cancer treatment information include preferably details on various cancer types, typical treatment methods (e.g., chemotherapy, radiation therapy, immunotherapy), potential side effects, and the frequency of certain treatments.

It is preferred that patient data is collected through a module via an user interface. By this, relevant data about the cancer patient is collected via a module (i.e., a software system or application) using a user interface (UI). This means preferably that the patient enters or selects information about their health status through an interactive interface.

The patient's health information includes, for example, personal data (e.g., name, age, gender), medical history (e.g., past illnesses, history of cancer or other diseases), current symptoms, treatment history, and medical results such as lab values or imaging procedures.

Cancer-related data includes preferably information about the cancer diagnosis, such as tumour type, stage, tumour size, specific genetic markers, or other relevant medical details that are important for treatment planning.

The user interface serves as a tool to collect this data easily and efficiently. It could be designed so that the patient answers questions or selects predefined options to facilitate data entry.

The goal of this step is to ensure a complete collection of relevant patient data, which can later be used for analysis, diagnosis, and further treatment planning. This helps ensure that the treatment is tailored to the individual needs of the patient.

Pseudonymization means that the patient data is modified so that it can no longer be directly associated with a specific person without additional knowledge. Identifying information, such as the name, address, or health insurance number, is replaced by pseudonyms (e.g., randomly generated identification numbers). The goal of pseudonymization is to protect privacy while ensuring that the data can still be used for research or treatment support purposes. It ensures that patient data cannot be traced back to a specific individual, safeguarding the patient's identity.

Pseudonymized data can still be used for scientific studies, analyses, or the development of treatment methods without compromising the patient's anonymity.

It is preferred that the patient data is stored on a server.

In this step, the pseudonymized patient data is preferably stored on a server, ensuring that the data is centrally stored and organized. The server serves as a secure location for the data, accessible to authorized individuals who can analyse or further process it.

The data is stored on a server (digital storage medium). A server is preferably a computer device or a group of computer devices designed to store and manage large amounts of data.

Since these are sensitive patient data, strict security measures are preferably implemented, such as encryption, access controls, and security protocols, to prevent unauthorized access. Only authorized individuals, such as medical professionals or researchers with appropriate permissions, can access these data.

Storing the data on a server ensures that all relevant information is centrally accessible and organized. This facilitates easy access and management of the data, regardless of the location or device used to access the information.

Patient data is stored in a secure location and can be efficiently retrieved and used. The server ensures that the stored data is protected from loss, theft, or unauthorized access, preserving patient privacy.

The patient data are analysed.

During this step, the pseudonymized patient data stored on the server undergo detailed analysis. Advantageously, the analysis can extract patterns, correlations, or relevant information from the data, which can be used to support treatment, treatment planning, and/or research.

Various analysis tools and/or algorithms can be used, such as artificial intelligence, statistical evaluations, and/or machine learning. These methods help to extract useful insights from the large data sets. The analysis may focus on identifying specific patterns, such as certain risk factors associated with the success or failure of a treatment. It can also help to evaluate the response to therapies or predict how the disease might progress for a particular patient. The insights gained can be used to help develop personalized treatment plans based on the patient's specific condition.

The analysis can also contribute to gain new insights in cancer research, leading to new therapeutic approaches or diagnostic methods. Additionally, early warning signs for relapses or complications can be identified, enabling early intervention.

A treatment recommendation is provided to the user interface to support the cancer patient's treatment.

In this step, based on the analysis of the patient data, a treatment recommendation is generated and made available to the patient through the user interface. This recommendation can assist the patient in understanding how promising the treatment may be. Specifically, the patient can advantageously recognize through the recommendation whether the treatment is promising for them and/or whether the treatment meets the guidelines for cancer treatment. The recommendation can serve as support for the patient's individual treatment, helping them determine if they may need to adjust the treatment and/or to change the oncologist. The recommendation is based on the analysed patient data, such as tumour type, stage of the cancer, genetic information, and previous treatment outcomes. It could include various therapeutic approaches, such as chemotherapy, radiation therapy, immunotherapy, or targeted therapies.

The treatment recommendation is displayed through a user-friendly interface, making it easy for the patient to understand and respond to. The interface could include a visual representation of the recommended treatment or a list of recommended options, along with corresponding notes and explanations.

This recommendation is not binding, but serves as a decision-support tool. The patient can consider it along with other medical factors, experiences, and personal judgment to decide on the next steps for treatment.

By providing a well-informed, data-driven recommendation, the decision-making process for the patient's treatment is simplified and optimized. The recommendation helps to tailor the treatment to the patient's specific needs, ensuring the best possible outcomes. The use of the user interface also allows treating physicians to quickly access relevant information and make more efficient treatment decisions in collaboration with the patient.

In a further preferred embodiment, analysing the patient data comprises an identification of an adherence to treatment guidelines, a detection of (rare) drug side effects and/or a comparison of an effectiveness of various treatment options.

The described preferred method step relates to the analysis of patient data in the context of supporting cancer treatment. Various aspects of the treatment can be reviewed. For example, it can be checked whether the patient's treatment complies with established medical guidelines. These guidelines are based on scientifically support and/or proven methods intended to guarantee the best possible success in cancer treatment.

Data can be analysed to identify potential side effects of the medications used, particularly those that are rare and may not be immediately recognized. This is advantageous in ensuring patient safety and detecting unwanted effects early.

An analysis can be conducted to compare the effectiveness of different therapeutic approaches. This checks which treatment is most promising for the specific patient, based on previous outcomes and the individual health status of the patient.

Treatment optimization can be achieved by verifying both adherence to standards and identifying potential risks and the best therapeutic option for the patient.

By identifying adherence to treatment guidelines and comparing various therapy options, the best possible treatment for the individual patient can be selected. This ensures a tailored therapy better suited to the patient's specific needs.

Particularly rare and difficult-to-recognize side effects of medications can be identified early. This allows timely measures to be taken to protect the patient from potential harm and adjust the treatment if necessary.

The continuous monitoring and analysis of patient data ensures that potential risks or adverse events are detected more quickly. This increases patient safety and can help avoid life-threatening complications.

Doctors can benefit from data-driven recommendations based on objective analysis. This reduces human error, facilitates decision-making, and helps select the best treatment approaches based on evidence and current data.

By comparing the effectiveness of different treatment options concerning the patient's specific health condition and genetic factors, treatments can be further individualized. This leads to better treatment outcomes.

In a further preferred embodiment, analysing the patient data comprise the following steps:
- data preparation and cleansing
- use of descriptive statistics
- use of survival analysis
- molecular biological data analysis
- correlation and regression analysis
- use of machine learning and predictive models
- multivariate analysis
- time series analysis
- presentation of results

The data preparation and cleansing ensure the quality of the data and prepare it for analysis. Data cleansing comprises preferably identifying and handling missing values, which can be addressed through imputation or deletion to ensure that the dataset remains complete and usable. Additionally, data standardization is carried out, where laboratory values and units are standardized to ensure consistency across different data sources and systems. Data integration is another preferred step, where clinical, molecular biological, and patient-specific data are merged to create a comprehensive profile of the patient's health and treatment history. Finally, outlier detection is preferably performed using statistical methods such as the Z-score or Tukey method to identify and manage extreme values that could skew the results. This step ensures that the data used for decision-making and analysis is accurate, consistent, and ready to provide valuable insights for personalized treatment plans and effective tracking of disease progression.

Descriptive statistics is preferably used to provide an overview of the data structure. The goal is to summarize and describe the main features of the data. This includes preferably analysing frequencies and distributions of various factors such as age, gender, and tumour stages to better understand the composition of the dataset. Additionally, preferably measures of central tendency and dispersion are calculated, including the mean, median, mode, and standard deviation, which help in understanding the overall distribution of the data. To make the data more accessible and interpretable, preferably visualizations such as boxplots, histograms, and scatter plots are created. These visual tools allow a clearer and better understanding of the data, highlighting patterns, trends, and potential outliers. The descriptive statistics can be helpful in analysing the patient data and/or identifying trends that can inform clinical decisions.

Survival analysis is preferably used to investigate the course of the disease and assess the success of the therapy. The goal is to understand how long patients survive after a particular treatment or diagnosis and which factors influence survival. One preferred method can the Kaplan-Meier analysis, which represents the survival curve and shows the probability of survival over time. To compare survival rates between different patient groups, preferably a Log-rank test is applied, helping to identify whether there are significant differences in survival between these groups. Additionally, preferably Cox regression, also known as the proportional hazards model, is used to analyse the impact of multiple variables on survival, such as treatment types, genetic factors, or other clinical conditions. The survival analysis can provide valuable insights into how the patient respond to different therapies, enabling a better personalization and better understanding of the disease progression.

The preferred molecular biological data analysis aims to explore the correlation between genetic markers and disease progression. This step preferably comprises gene expression analysis, which compares the expression levels of genes between patients who have different disease courses. This can be done using techniques such as RNA-Seq data. Differential gene expression analysis (DGE) can also be performed to identify significant differences in gene expression, often through statistical methods like t-tests or ANOVA. Another preferred step of the molecular biological data analysis is cluster analysis, which helps to identify subgroups of patients based on their molecular biology profiles. Techniques such as hierarchical clustering, including methods like Ward's, and k-means clustering are used to group patients with similar genetic patterns. The molecular biological data analysis can uncover valuable insights into how genetic factors influence disease progression and support the development of personalized treatment strategies tailored to the molecular characteristics of the patient's cancer.

Correlation and regression analyses are used to examine the relationship between clinical parameters and disease progression. The goal is to identify how different factors, such as age, gender, or treatment types, influence the course of the disease. Pearson correlation is employed to assess linear relationships between variables, helping to understand whether changes in one parameter are associated with changes in another. Additionally, multiple linear regression is used to analyse the impact of several influencing factors on disease progression simultaneously. This method helps to identify which factors are most significant in predicting how the disease advances. In a software tool for cancer patients, these analyses can provide insights into the key clinical factors that affect outcomes, enabling more accurate predictions and personalized treatment strategies.

The preferred correlation and regression analyses are used to examine the relationship between clinical parameters and disease progression. The goal is to identify how different factors, such as age, gender, or treatment types, influence the course of the disease. Pearson correlation is preferably employed to assess linear relationships between variables, helping to understand whether changes in one parameter are associated with changes in another. Additionally, multiple linear regression is preferably used to analyse the impact of several influencing factors on disease progression simultaneously. This helps to identify which factors are most significant in predicting how the disease advances. This step can provide insights into the key clinical factors that affect outcomes, enabling more accurate predictions and personalized treatment suggestions.

The preferred use of machine learning and predictive models can help to predict disease progression and therapy response. The goal is to develop models that can forecast how a patient's disease will progress and how they will respond to treatment based on historical data. Classification models, such as random forest, support vector machines (SVM), and neural networks, can be used to categorize and/or predict outcomes. To ensure the reliability and robustness of these models, cross-validation is preferably performed, which involves testing the model on different subsets of the data to assess its performance. The quality of the model is further preferably evaluated using ROC curves (Receiver Operating Characteristic) and AUC values (Area Under Curve), which help determine the model's ability to correctly classify patients and make accurate predictions.

Multivariate analyses are preferably used to investigate complex relationships within the data. The goal is to explore how multiple variables interact and contribute to disease progression. One preferred technique is principal component analysis (PCA), which reduces the number of dimensions in molecular data, making it easier to interpret and visualize. PCA helps to identify the most important features that explain the variability in the data. Eigenvalue decomposition of a covariance matrix is preferably used to identify patterns and relationships between different variables. Factor analysis is another method that can help to analyses the underlying structure of symptoms and molecular markers, helping to uncover latent factors that influence the disease. Multivariate analyses can provide a deeper understanding of how various factors, including molecular data, clinical parameters, and symptoms, are interrelated, enabling more comprehensive and personalized support options for the patient regarding the treatment.

Time series analyses can be used to examine the progression of disease parameters, such as tumour markers, over time. The goal is to understand how these parameters change and evolve, which can provide insights into disease progression and treatment effectiveness. Autoregressive models (AR) are used to predict future values based on past data, capturing the relationships between current and past observations. Additionally, ARIMA models (Autoregressive Integrated Moving Average) are preferably applied for forecasting, as they combine autoregressive and moving average components to account for trends and seasonal variations in the data. Time series analysis can help track the dynamic changes in disease markers over time, enabling more accurate predictions and personalized treatment adjustments based on the evolving condition of the patient.

The presentation of results (and interpretation) is advantageous for effectively communicating the findings from the analysis. This comprises preferably generating reports and dashboards that visualize the data through graphs, heat maps, and interactive diagrams, making it easier to understand complex patterns and trends. The significance level of the results is preferably determined through one or more p-value calculations, which help to assess whether the observed effects are statistically significant. Preferably, additionally confidence intervals are calculated to provide a range of values within which the true result is likely to lie, offering a measure of the precision of the estimates. These visualizations and statistical measures help to interpret the data accurately, leading to better-informed decisions and treatment plans and adjustments for the patient, the doctors, particularly the oncologist, and/or researchers.

The combination of classical statistics, multivariate methods, and machine learning provides a comprehensive approach to analysing disease progression. By integrating these techniques, it is possible to gain deeper insights into how different factors influence the course of the disease. Data-driven models are especially valuable when incorporating molecular biological data, as they can predict how a patient will respond to therapy and help develop personalized treatment strategies.

The integration of classical statistics, multivariate methods, and machine learning offers a powerful and holistic approach to analysing disease progression, especially in cancer patients. Classical statistics provide essential tools for understanding the data's fundamental characteristics, such as distributions, central tendencies, and variability. Multivariate methods allow for the examination of complex relationships between multiple variables, helping to identify patterns and interactions that may not be immediately apparent. Machine learning, on the other hand, enables the development of predictive models that can adapt and learn from vast datasets, providing highly accurate forecasts for disease progression and therapy response.

The combination of these techniques is particularly valuable when molecular biological data is incorporated. This allows for the exploration of genetic markers, gene expressions, and other molecular factors that influence how the disease evolves and how a patient responds to various treatments. By leveraging data-driven models, a patient's likely response to different therapies can be predicted, providing clinicians with crucial insights for developing personalized treatment strategies. These personalized approaches are designed to be more effective because they are tailored to the specific characteristics of the patient's disease, improving treatment outcomes and minimizing unnecessary side effects.

Overall, these preferred steps for analysing not only aid in understanding the complexity of the cancer disease but also support the development of precision medicine, ensuring that each patient receives the most appropriate and effective treatment (or treatment recommendation) for their unique condition. The combination of advanced statistical methods, machine learning, and molecular data integration is very advantageous in enhancing cancer care and improving patient survival rates.

In a further preferred embodiment, the analysis is carried out by an artificial intelligence, preferably by a machine learning model, wherein preferably the machine learning model is based on supervised learning, unsupervised learning, and/or reinforcement learning, wherein preferably the supervised learning is selected from the group consisting of logistic regression, SVM (Support Vector Machines), Naive Bayes, decision trees, linear regression, kNN (K-nearest neighbours), random forest, and boosting algorithms, and wherein preferably the unsupervised learning is selected from a group comprising a k-means algorithm and/or hierarchical clustering

The preferred method uses artificial intelligence (AI), specifically machine learning, to support the analysis of patient data in cancer treatment. Machine learning enables the identification of patterns in large data sets and makes predictions without the need for explicit programming. This is particularly useful in cancer treatment as it helps to analyse individual patient data. Within the context of this method, different learning approaches can be used.

In supervised learning, the model is trained with pre-existing data that includes both input values (e.g., patient data) and corresponding output values (e.g., the body's response to a treatment). The goal is for the model to learn from these examples and then make predictions for unknown data. Common algorithms used in this approach include logistic regression, which is primarily a classification method that calculates the likelihood that a patient will respond to a specific treatment based on various characteristics.

Support Vector Machines (SVM) is another powerful classifier often used to separate data points into different categories; in cancer treatment, it helps distinguish between various types or stages of cancer.

Naive Bayes, a probabilistic classifier, is also commonly used to identify patterns in large data sets and estimate how likely a specific treatment outcome is.

Decision trees are models that use decision rules based on patient data to make predictions, offering the advantage of visually understandable decision processes.

Linear regression is typically used to determine continuous values, such as the duration of treatment effectiveness or the progression of side effects.

K-nearest neighbours (KNN) works by identifying patients with similar characteristics, such as genetic information or reactions to previous treatments.

Random forest is an ensemble algorithm that consists of many decision trees, leading to more robust predictions.

Boosting algorithms combine multiple weak learning algorithms to create a strong model, and these can be used to calculate the probability of treatment success in different scenarios.

In unsupervised learning, no predefined output values are used. Instead, the model searches for patterns or structures in the input data. This approach is particularly useful in cancer treatment as it helps discover new, unknown patterns or correlations that could lead to better treatment strategies.

One example is the k-means algorithm, a clustering method that groups patients based on similar features. This could be used, for example, to group patients with similar disease progressions and determine the most effective treatment based on that information.

Another example is hierarchical clustering, which also divides patients into clusters, and can be helpful in identifying different subtypes of cancer or varying responses to treatment methods.

Reinforcement learning involves training a model by rewarding or punishing it through interactions with its environment, such as the patient. In cancer treatment, this could be used to develop the optimal treatment strategy in real-time. Through continuous learning, the model can make the best decisions for the patient, such as determining the most effective combination of drugs or therapies during the course of treatment.

In a further preferred embodiment, the treatment recommendation comprises one or more of guideline-based treatment recommendations, connections to patient support groups, information on therapies undergoing clinical trials, and/or access to verified information from artificial intelligence applications and medical platforms.

Guideline-based treatment recommendations are based on current, scientifically supported treatment guidelines and medical standards developed by recognized professional societies and organizations. They provide evidence-based guidance and help in selecting the best possible therapy for the patient.

Connecting patients to support groups is beneficial, as interacting with others who are affected by the same condition can be an important source of support. The patient is linked to self-help groups or support networks that offer emotional support and share experiences and information on coping with the disease and treatment.

The patient is provided with information about clinical trials in which new therapies or treatment methods are being tested. These trials may offer patients access to innovative treatments that are not yet widely available and can increase the chances of successful treatment.

Access to verified information from artificial intelligence (AI) and medical platforms primarily refers to accessing verified, Al-assisted information provided by medical platforms. AI can assist in analysing relevant data and offering personalized, evidence-based recommendations based on the latest scientific findings and patient data.

By incorporating guideline-based recommendations, it is ensured that the patient's treatment is based on the latest scientific knowledge and recognized standards. This increases the chances of therapy success and minimizes risks. Access to clinical trials provides the patient with the opportunity to participate in innovative treatments that could be individually beneficial, especially if conventional therapies do not yield the desired results. Contact with support groups helps alleviate the emotional pressure that cancer can place on the patient. Sharing experiences with others who are affected offers valuable support and eases the process of coping with the illness. Access to Al-supported information ensures that the patient receives reliable and always up-to-date information. Al systems can analyse large data sets and provide personalized, targeted recommendations tailored to the individual situation of the patient. Access to a wide range of information sources expands the treatment options available to the patient, allowing them to consider different possibilities and select the most suitable therapy. By providing comprehensive information, the patient is involved in the decision-making process and can actively participate in their treatment. This promotes a sense of control and empowerment, which can positively affect the treatment outcome.

In a further preferred embodiment, the interface comprises a chatbot (or chatbot interface) configured to answer patient queries and/or deliver personalized health updates.

A chatbot is preferably an Al-driven program that can communicate with the patient via text and/or voice to provide information, answer questions, and assist the patient with their treatment.

The chatbot interface allows patients to interact at any time with an automated, Al-powered application to get answers to their questions. These questions can relate to various topics, such as information about treatment options, medication side effects, tips for symptom relief, and/or details about scheduled doctor appointments.

In addition to answering questions, it may be preferable for the chatbot to deliver personalized health updates to the patient. This could include regular updates on the course of the treatment, reminders for appointments or medication instructions, as well as adjustments to the treatment based on the collected patient data.

The chatbot is advantageously available around the clock, so the patient can get answers to their questions and/or information about their treatment at any time, without relying on office hours of doctors or clinic staff. This ensures continuous care and increases the accessibility of information.

Chatbots can answer queries in real-time, which is beneficial for quickly resolving issues and facilitating decision-making. The patient does not have to wait for answers from a doctor or healthcare provider, which can be especially helpful in stressful or uncertain times.

The chatbot is advantageously able to provide personalized information based on the patient's specific health data and treatment history. This means that the patient receives not only general information but also details tailored to their individual situation. This fosters more accurate and relevant communication.

Direct and interactive communication with the chatbot encourages patient engagement. By regularly interacting with the chatbot, the patient stays better informed and more active in their own treatment, which can lead to better adherence to the treatment plan and higher patient satisfaction.

Since the chatbot can handle simple inquiries and routine updates independently, healthcare staff is relieved. Doctors and nurses do not need to spend time addressing routine questions and can focus their time on more complex or specific issues that require human interaction.

The chatbot can present information in a clear and standardized way, minimizing misunderstandings due to unclear communication or stressful situations (e.g., in a clinical setting). This ensures that the patient receives the correct instructions and that no important information is overlooked.

Overall, a preferred chatbot enhances the availability of information, promotes treatment adherence, and enables personalized care while also relieving healthcare staff. This ultimately leads to better patient care and a more positive treatment experience.

In a further preferred embodiment, wherein the patient data comprise personal data and disease-related data, wherein preferably the personal data are selected from one or more of the group consisting of demographic data, such as age, gender, body height, place of residence, profession, and other data, such as dietary habits, the name of the doctor currently treating the patient, wherein preferably the disease-related data are selected from one or more of the group consisting of histological diagnostic data, molecular diagnostic data, tumour classifications, medication intake, reactions to medication intake, therapies, treatment recommendations, diagnostic imaging data, residual status, performance status.

In other words, it may be preferred that patient data is analysed and evaluated to support personalized treatment options for cancer patients. Patient data preferably includes information about the patient that is used to create a specific treatment plan. This data can preferably be divided into two main categories: personal data and disease-related data.

Personal data primarily refers to general information about the patient that is not directly related to the disease but may still be relevant for treatment. Examples include demographic data such as age, gender, body height, place of residence, occupation, and other data such as dietary habits or the name of the treating doctor.

Disease-related data primarily refers to information directly related to the patient's cancer. Disease-related data preferably includes histological diagnostic data (tissue samples that provide information about the tumour), molecular diagnostic data (genetic information that helps to characterize the cancer more precisely), tumour classifications (information about the type of cancer, e.g. cancer stage), medication intake (which medications the patient is taking and how they are responding), therapies and treatment recommendations (information about therapies performed and recommended next steps), diagnostic imaging data (images from imaging techniques such as CT, MRI, or X-rays that show the progression of the disease), residual status and performance status (the current condition of the patient and their ability to perform daily activities).

By collecting and analysing both personal and disease-related data in detail, an individualized treatment strategy tailored to the patient can be developed. This ensures that treatments are better aligned with the patient's needs and condition. By integrating disease-related data such as medication reactions or performance status, the procedure can help detect undesirable reactions or complications early and respond quickly. The use of histological and molecular diagnostic data leads to a more accurate diagnosis of cancer, which can result in a better assessment of the disease stage and a more targeted treatment. The procedure can assist doctors in decision-making by presenting relevant data in a structured way, thus reducing the time needed to analyse and collect information. This saves valuable time and allows for a quicker response to changes in the patient's condition.

In a further preferred embodiment, the histological diagnostic data comprises one or more of histological grading, immunohistochemistry (IHC) and in situ hybridization (ISH).

Histological diagnostic data preferably include information obtained from the examination of tissue samples. In histology, tissue is examined under the microscope to analyse characteristics of cancer cells. The histological data encompass various methods to determine the type, stage, and behaviour of a tumour.

Histological diagnostic data preferably include histological grading. This refers to the assessment of tumour cells based on their appearance under the microscope. Grading indicates how much the tumour cells differ from healthy cells and how aggressive the tumour is likely to be. A higher grade indicates a more aggressive, faster-growing tumour.

Histological data preferably also include immunohistochemistry (IHC). In immunohistochemistry, a special technique is used in which antibodies are applied to detect specific proteins in tumour cells. These proteins can be specific markers that determine the type of tumour, its aggressiveness, or its response to certain treatments. IHC is often used to determine whether a tumour expresses certain receptors or molecules, which can be crucial in selecting targeted therapies (e.g., hormone receptors in breast cancer).

Histological data preferably also include in situ hybridization (ISH). In situ hybridization is primarily used to detect specific RNA or DNA sequences in the cells. ISH allows the identification of genetic changes or specific markers at the molecular level that indicate the presence or behaviour of the tumour. For example, ISH can be used to detect genetic mutations that influence the tumour.

By incorporating histological grading, IHC, and ISH, tumours can be characterized more accurately. This helps to better understand the specific behaviour of the tumour, leading to a more precise diagnosis. Histological grading provides an assessment of the tumour's aggressiveness. This can determine whether a more aggressive or less intensive treatment is necessary. Immunohistochemistry (IHC) reveals whether the tumour expresses certain molecules or receptors. This information is crucial in selecting targeted therapies, such as the use of hormone therapies for hormone-sensitive tumours or monoclonal antibodies for certain types of cancer.

With in situ hybridization (ISH), genetic mutations or specific markers of the tumour can be identified. This helps to understand the tumour at the molecular level and develop a personalized therapy that is precisely tailored to the genetic characteristics of the tumour.

Through comprehensive molecular and histological insight into the tumour, doctors can make an informed decision about how the treatment should proceed. The information enables a more accurate assessment of the patient's prognosis and the development of the best treatment plan.

If tutor cells exhibit specific proteins or genetic characteristics associated with resistance to certain medications, these can be detected early. This allows for the quick adjustment of treatment to ensure effective therapies.

In a further preferred embodiment, the molecular diagnostic data comprise one or more of next-generation sequencing (NGS) data and microarray data.

NGS, also known as next-generation sequencing, preferably refers to a sequencing technology that allows for the complete or partial sequencing of a tumour's DNA. NGS enables the simultaneous analysis of millions of DNA strands, thereby detecting genetic changes (such as mutations, deletions, or amplifications) associated with the development and progression of cancer. In the context of cancer diagnosis, NGS can preferably be used to identify the genetic signature of a tumour. This allows for the selection of targeted therapies aimed at specific genetic changes in the tumour (e.g., in the treatment of lung cancer with EGFR mutations or breast cancer with HER2 overexpression).

Microarray technology preferably refers to a method in which thousands of genes are examined simultaneously on a small glass or silicon chip. By analysing gene expression (i.e., which genes are active in the tumour cells), researchers and doctors can identify patterns of gene activity that indicate specific types or stages of cancer. Microarrays are preferably used to measure gene expression and thus gain insights into tumour behaviour and prognosis. For example, they can help classify tumours based on their molecular signature or identify genetic markers associated with poor prognosis or resistance to certain treatments.

With NGS, tumours can preferably be studied at the DNA level to detect precise genetic mutations or alterations associated with the tumour's development and behaviour. This genetic information provides a more accurate diagnosis and helps to better understand the nature of the cancer. NGS enables the identification of specific mutations in the tumour that can be targeted with specific medications. In tumours with genetic changes such as EGFR mutations or ALK translocations, targeted therapies (such as tyrosine kinase inhibitors) can be used that specifically target these mutations.

Microarray data preferably provide detailed information about gene expression and allow for the classification of tumours based on their molecular signature. This improves diagnostic accuracy and helps differentiate tumours from similar conditions. Microarrays can also be advantageously used to identify tumour markers that are crucial for selecting targeted therapies. One example is the identification of tumours with HER2 overexpression in breast cancer, for which trastuzumab can be used as a targeted therapy.

The genetic information from NGS and microarrays enables optimized treatment planning. Since they characterize the tumour at the molecular level, less invasive but more effective treatment options can be selected. This increases the likelihood of a positive treatment outcome and minimizes unnecessary or ineffective treatments.

In a further preferred embodiment, the diagnostic imaging data comprise one or more data selected from the group consisting of computed tomography (CT), scintigraphy, magnetic resonance imaging (MRI), and positron emission tomography-computed tomography (PET-CT).

Computed tomography (CT) is preferably an imaging method in which X-rays are directed at the body from various angles. These rays are captured by a detector and processed by a computer into detailed cross-sectional images (slices) of the body. CT is particularly well-suited for detecting tumours in various regions of the body, determining their size and location, and visualizing metastases (secondary growths). CT is preferably used for the planning of surgical interventions or radiation therapies, as it provides detailed information about tumour structures.

Scintigraphy refers to a preferred procedure in nuclear medicine imaging, where radioactive substances (radiopharmaceuticals) are injected into the body. These substances accumulate in specific tissues and emit radiation, which is captured by a camera. Scintigraphy can be used to identify tumours and assess their function, especially in certain types of cancer, such as thyroid cancer or bone metastases. It provides information about the activity of tumours by making the metabolism and function of the cells visible.

Magnetic resonance imaging (MRI) uses strong magnetic fields and radio waves to create detailed images of the body's internal structures. MRI is often used to visualize soft tissue tumours (such as those in the brain, liver, or internal organs) as it offers particularly good soft tissue contrast. Unlike CT, MRI does not use X-rays. MRI can also be preferably used to measure the extent of tumours and assess their relationship to adjacent tissues.

Positron emission tomography-computed tomography (PET-CT) preferably combines positron emission tomography (PET) with computed tomography (CT). In PET, radioactive markers are used to represent metabolic activities (metabolic processes) in the body, while CT provides structural images of the body. The combination of these two methods provides both functional (metabolic) and anatomical (structural) information and is particularly useful in assessing tumours. PET-CT can help detect tumours early, identify metastases, and monitor the effectiveness of treatments by visualizing tumours based on their metabolic activity.

CT, MRI, scintigraphy, and PET-CT offer an extremely precise method for early detection of tumours. In particular, PET-CT can make tumours visible based on their metabolic activity at an early stage, even before they are structurally detectable, leading to better early detection and thus a higher success rate in treatment.

With the help of CT, MRI, and PET-CT, tumours can be accurately located, and their stage (staging) can be determined. This means that doctors can assess the tumour's size, exact location, and any metastases in the body. This information is crucial for planning a targeted and effective treatment, such as precise radiation therapy or surgical removal of the tumour.

PET-CT is particularly helpful in identifying metastases as it visualizes tumours based on their increased metabolic activity. This helps detect secondary tumours (metastases) early, even when they are still too small to be visible on traditional CT or MRI.

In a further preferred embodiment, the server is secured and/or the transmission of patient data to the server and from the server is encrypted.

The security of the server refers to technical and organizational measures taken to protect the server, on which patient data is stored, from unauthorized access, data loss, or other security threats. This includes, for example, firewalls, security software, and access controls that ensure only authorized individuals can access the server.

Encryption is the process of converting information into an unreadable form that can only be decrypted by authorized recipients. Encryption of data transmission refers to the practice of encrypting data sent or received between the server and other devices (such as a doctor's computer or a patient's device). This ensures that, even if the transmission is intercepted, the data cannot be read by third parties. Encryption is preferably performed using special algorithms that ensure that only authorized parties with the correct decryption key can revert the data to its original form.

The encryption of data transmission and the security of the server ensure that confidential cancer patient information (such as medical diagnoses, treatment histories, and/or genetic data) is protected from unauthorized access. This is especially important as patient data contains sensitive personal information, the unauthorized disclosure of which could have serious consequences for the privacy and security of the patients.

In many countries, there are strict legal regulations regarding data protection, particularly in healthcare (e.g., the General Data Protection Regulation (GDPR) in Europe). The encryption of data transmission and the security of the server help comply with these regulations and ensure that patient data is processed and stored only in accordance with legal requirements.

Ensuring secure communication and data storage helps healthcare institutions avoid legal penalties or liabilities due to data protection violations.

By securing the server and encrypting data, unauthorized third parties, such as hackers or unauthorized staff, are prevented from accessing patient data. In the context of cancer treatment, misused or stolen data could have serious consequences for the patient and the treatment, such as identity theft or manipulation of medical data.

When patients know that their medical data is securely stored on an encrypted server and transmitted via secure channels, it strengthens their trust in the healthcare system and treatment facilities. This trust is particularly important when it comes to sensitive and personal information, such as cancer treatment.

Server security ensures that the data is protected not only from unauthorized access but also from loss or damage. Patient records, test results, and treatment plans can always be accurately and safely retrieved. The integrity of the data is crucial to ensuring the correct diagnosis and treatment.

Encryption prevents third parties, who might illegally intrude into communication, from accessing the transmitted data. This reduces the risk of data leaks that could arise from insecure transmission paths or inadequate server security measures.

In modern treatment approaches for cancer patients, telemedicine services or remote monitoring tools can be used to provide care to patients from a distance. The encryption of data transmission ensures that diagnostic results, treatment recommendations, or even monitoring data (e.g., vital signs) can be securely exchanged between the patient and the treating physicians without third parties being able to view the information.

When patient data is securely encrypted and stored on a protected server, it also minimizes the risk of that data being manipulated afterward. Unauthorized changes to medical data could lead to incorrect treatment decisions, which could have life-threatening consequences, especially in the treatment of cancer patients.

In a further preferred embodiment, an unique pseudonymized identification code is generated and assigned to the patient.

A unique pseudonymized identification code preferably refers to a unique identification code and is preferably a digital key assigned to a specific patient. This code is preferably pseudonymized, meaning it does not contain directly identifiable information (such as name or date of birth) but instead represents an anonymized key that uniquely identifies the patient without revealing their personal data.

The code is created using an algorithm or program logic and assigned to the patient. This process ensures that each patient receives an individual code that protects their anonymity.

The pseudonymized code safeguards the privacy of patients by protecting their personal data from unauthorized access. This is particularly important when it comes to sensitive health data. For example, medical data can be stored without directly accessing the patient's identity.

By using a pseudonymized code, misuse and discrimination can be avoided, as the code does not disclose any direct personal information. This is especially important in the medical context, where discretion and trust are required.

In a further preferred embodiment, the identification code has already been assigned by a software system of the doctor currently treating the patient.

The identification code is, as described above, preferably a unique identifier for the patient. This could, for example, be a number or a digital key that uniquely identifies the patient for all interactions.

"Already been assigned by a software system of the doctor" preferably means that the identification code has already been assigned by the software system of the doctor currently treating the patient. This preferably means that the doctor, based on their own medical system, has assigned the code before the patient uses the preferred method. This ensures that the patient's medical information is consistent and accurate.

The doctor who has assigned the identification code is preferably also the one currently responsible for the patient's treatment. Therefore, the code is created by the doctor who is in charge of the patient, and there is a direct connection between the preferred method and the treating doctor.

When the identification code has already been assigned by the treating doctor, it ensures that the patient data stored in the preferred method is accurate and up-to-date. This reduces the possibility of errors or confusion and ensures that all information regarding the patient's treatment comes from a trusted source. The code assigned by the treating doctor ensures a close link between the preferred method and the current treatment of the patient. This facilitates coordinated care and ensures that all parties, both doctors and patients, use the same dataset. Since the code is assigned by the treating doctor and not manually by the patient or another source, the risk of duplicates (patients with multiple profiles) or inconsistencies in the data is reduced. The code ensures that each patient is uniquely and correctly identified based on the doctor's information.

The assignment of the identification code through the doctor's software system enables seamless communication and data exchange between the preferred method and the doctor's medical systems. The patient does not have to worry about the assignment themselves, as everything has been set up for them. Since the identification code has already been created by the doctor's software system, the patient could directly access their treatment history when opening the preferred method without needing additional identification steps. This saves time and reduces potential sources of error.

Patients and doctors can be confident that the preferred method is reliable with identification codes assigned by a professional system. This increases the patients' trust, as they know their health data has been validated by the treating doctor and not just by generic software.

In a further preferred embodiment, information of a medical report is stored on the server and/or considered during the analysis.

Information from a medical report preferably includes the information contained in a medical report. A medical report may include various data, such as diagnoses, treatment plans, test results, images from examinations (such as CT scans or MRIs), or notes from the treating physician. This information is crucial for monitoring and analysing a patient's health condition.

This information is preferably stored on a server, meaning it is centrally stored on a computer device or group of computer devices and/or in a cloud. The server acts as a secure database that authorized healthcare professionals can access to retrieve or edit the relevant information. Storing the information on a server allows for easy data management and backup.

"And/or considered during the analysis" here preferably means that, in addition to being stored on the server, the stored information may also be considered during the analysis of the patient. This preferably means that this medical data can be incorporated into evaluations and recommendations to provide the patient with targeted support in their treatment. The analysis could, for example, include the evaluation of treatment outcomes, medication interactions, or the progression of the patient's health condition.

Storing the medical reports on a server advantageously ensures that all relevant information is collected in a secure and central location. This simplifies access to the data, both for the patient and for doctors or other healthcare providers. The patient no longer needs to manually collect or submit reports but can have everything readily available in the preferred method and the associated systems.

When the medical reports are stored on a server, a better care for the patient can be achieved. A direct connection is created between the method, the treating doctor, and other medical professionals. This leads to better coordination of care and more informed decision-making.

When the medical data is considered during the analysis, the preferred method can provide personalized recommendations and support. For example, based on test results or diagnoses, it could offer tailored information or treatment advice. This personalized care is essential, especially in the treatment of cancer patients, where each treatment must be individually tailored.

The preferred method could use automated analysis processes to detect important trends or patterns from the stored medical reports. For example, it could identify changes in the patient's health condition (such as tumour growth or side effects of a therapy) at an early stage and notify the patient or medical team.

By storing all medical reports and integrating analysis into the preferred method, the patient's progress during treatment can be easily tracked. This can provide a history of treatments, tests, and results, which is highly valuable for evaluating the success of the treatment and adjusting the therapy.

In a further preferred embodiment, the data collection is configured for real-time integration with national or international cancer treatment registries.

Data collection preferably refers to the process of gathering relevant data about the patient and their treatment. This data can include a variety of information, such as diagnoses, test results, treatment methods, medication intake, or clinical outcomes. The data collection is carried out by automatically or manually, based on the interactions and information the patient provides.

"Configured for real-time integration" preferably means that the collected data is not only stored but can also be transmitted in real time to other systems or databases. In this case, real-time integration is designed so that the data flows immediately into other relevant systems without delays or manual steps.

"National or international cancer treatment registries" preferably refer to official databases where information about cancer treatments, diagnoses, and disease progressions is stored. These registries can exist on a national level (e.g., in a specific country) or internationally (global exchange of treatment data). They serve to collect and analyse treatment outcomes and promote research, therapy optimization, and epidemiological studies.

Through real-time integration, the collected patient data can be transmitted immediately to national or international cancer treatment registries. This enables the rapid and continuous collection of treatment data that benefits both the treating physician and the global medical community.

The integration of patient data into national or international cancer registries contributes to the improvement of cancer research. Researchers and doctors can access anonymized treatment data to identify patterns, develop new treatment methods, and better understand the overall course of cancer diseases.

A connection in real-time to national or international registries, an alignment with the latest treatment recommendations and guidelines can be established. This ensures that patients receive treatment that aligns with current scientific findings and best practices, and that their treatment is continuously monitored to ensure it matches the latest research.

Direct integration into cancer registries ensures that all collected treatment data is accurately and completely recorded. This data then becomes part of a larger database, enabling comprehensive analysis and accurate evaluation of treatment outcomes.

By matching the collected data with the registries, potential errors or discrepancies can be detected more quickly. For example, unusual treatment approaches or side effects could be immediately reported and reviewed, allowing for a faster response to health issues and enhancing patient safety.

Real-time data integration ensures that the progress of treatment and the development of the patient's health condition are constantly monitored. This leads to a quicker adjustment of therapy if necessary and allows the medical team to continuously optimize the treatment.

A real-time feedback to doctors on the course of treatment and the collected data can be provided. This data could contribute to better decision-making and help develop more personalized and effective treatment methods based on the latest trends and treatment data.

Through integration with international cancer registries, patient treatment data becomes accessible on a global scale. This promotes international collaboration in cancer research and treatment and allows for benefiting from the best global practices and utilizing a broader range of experience.

The continuous collection and integration of treatment data from various sources can help identify long-term patterns that may be relevant for preventive measures or early detection strategies. This could lead to better forecasts for future patients and contribute to the development of preventive therapies.

In a further preferred embodiment, the cancer is selected from the group consisting of lung cancer, breast cancer, colorectal cancer, prostate cancer, cervical cancer, bladder cancer, head and neck cancer, oesophageal cancer, melanoma, lymphoma

These types of cancer represent different kinds of cancer that can be treated or tracked in the preferred method. Each of these cancers has its own treatment approaches, specific risk factors, and therapy options, which require an individualized approach.

Since the claim includes a selection of specific cancers, the preferred method can tailor the treatment plan to the specific type of cancer. For each of the mentioned cancers, there are different treatment options, therapies, and prognoses, which are considered to provide patients with the best possible personalized care.

For each of the listed cancers, there are extensive clinical data and treatment recommendations that can be integrated into the preferred method. This ensures that patients can access evidence-based treatment methods that are proven to be effective, and supports both doctors and patients in selecting the best treatment.

The user interface could also point patients with a specific type of cancer to appropriate clinical trials relevant to their diagnosis. With many cancers listed, there are constantly new studies, that can help patients explore these opportunities.

For each of the mentioned cancers, specific information about the disease, treatment, and follow-up care could be provided. This helps patients become better informed about their condition, leading to better collaboration with the medical team and a deeper understanding of their health status.

Since different cancers require different treatment teams, a collaboration between various specialists can be promoted. For example, a cancer patient might consult a pulmonologist, an oncologist, and a surgeon. The preferred method would allow all relevant information to be shared in real-time, ensuring coordinated and comprehensive care.

In a further aspect, the invention relates to a computer program product comprising instructions which, when the program is executed by a computer, cause the computer to carry out the steps of the computer-implemented method according to the invention or any one of the preferred embodiments.

The person skilled in the art knows that the features, definitions, and advantages of the computer-implemented method according to the invention, as well as of preferred embodiments of the computer-implemented method, also apply to the computer program product, and vice versa.

## Claims

1. A computer-implemented method for supporting a treatment of a cancer patient comprising the following steps:
• Providing information on cancer treatment and cancer treatment guidelines to the cancer patient,
• collecting patient data on a module via an user interface during the treatment,
• pseudonymizing the patient data,
• storing the patient data on a server,
• analysing the patient data,
• providing a treatment recommendation at the user interface to support the treatment of the cancer patient.

2. Computer-implemented method according to claim 1
wherein analysing the patient data comprises an identification of an adherence to treatment guidelines, a detection of (rare) drug side effects and/or a comparison of an effectiveness of various treatment options.

3. Computer-implemented method according to any one of the preceding claims, wherein analysing the patient data comprise the following steps:
- Data preparation and cleansing
- use of descriptive statistics
- use of survival analysis
- molecular biological data analysis
- correlation and regression analysis
- use of machine learning and predictive models
- multivariate analysis
- time series analysis
- presentation of results.

4. Computer-implemented method according to any one of the preceding claims,
wherein the analysis is carried out by an artificial intelligence, preferably by a machine learning model, wherein preferably the machine learning model is based on supervised learning, unsupervised learning, and/or reinforcement learning, wherein preferably the supervised learning is selected from the group consisting of logistic regression, SVM (Support Vector Machines), Naive Bayes, decision trees, linear regression, kNN, random forest, and boosting algorithms, and wherein preferably the unsupervised learning is selected from a group comprising a k-means algorithm and/or hierarchical clustering.

5. Computer-implemented method according to any one of the preceding claims,
wherein that the treatment recommendation comprises one or more of guideline-based treatment recommendations, connections to patient support groups, information on therapies undergoing clinical trials, and/or access to verified information from artificial intelligence applications and medical platforms.

6. Computer-implemented method according to any one of the preceding claims,
wherein the interface comprises a chatbot interface configured to answer patient queries and/or deliver personalized health updates.

7. Computer-implemented method according to any one of the preceding claims,
wherein the patient data comprise personal data and disease-related data,
wherein preferably the personal data are selected from one or more of the group consisting of demographic data, such as age, gender, body height, place of residence, profession, and other data, such as dietary habits, the name of the doctor currently treating the patient,
wherein preferably the disease-related data are selected from one or more of the group consisting of histological diagnostic data, molecular diagnostic data, tumour classifications, medication intake, reactions to medication intake, therapies, treatment recommendations, diagnostic imaging data, residual status, performance status,
wherein preferably the histological diagnostic data comprises one or more of histological grading, immunohistochemistry (IHC) and *in situ* hybridization (ISH).

8. Computer-implemented method according to claim 7, wherein the molecular diagnostic data comprise one or more of next-generation sequencing (NGS) data and microarray data.

9. Computer-implemented method according to claim 7, wherein the diagnostic imaging data comprise one or more data selected from the group consisting of computed tomography (CT), scintigraphy, magnetic resonance imaging (MRI), and positron emission tomography-computed tomography (PET-CT).

10. Computer-implemented method according to any one of the preceding claims, wherein the server is secured and/or the transmission of patient data to the server and from the server is encrypted.

11. Computer-implemented method according to any one of the preceding claims
wherein a unique pseudonymized identification code is generated and assigned to the patient
and/or the identification code has already been assigned by a software system of the doctor currently treating the patient.

12. Computer-implemented method according to any one of the preceding claims
wherein information of a medical report is stored on the server and/or considered during the analysis.

13. Computer-implemented method according to any one of the preceding claims
wherein the data collection is configured for real-time integration with national or international cancer treatment registries.

14. Computer-implemented method according to any one of the preceding claims wherein the cancer is selected from the group consisting of lung cancer, breast cancer, colorectal cancer, prostate cancer, cervical cancer, bladder cancer, head and neck cancer, oesophageal cancer, melanoma and/or lymphoma.

15. Computer program product comprising instructions which, when the program is executed by a computer, cause the computer to carry out the steps of the computer-implemented method of any one of claims 1 to 14.
